# EUROPEAN PATENT APPLICATION

(11) **EP 1 155 699 A1**
(43) Date of publication of application: **21.11.2001**
(21) Application number: 01111751.2
(22) Date of filing: 15.05.2001
(51) Int. Cl.: A61K 31/714, A61K 31/575, A61K 31/519, A61K 31/4415, A23L 1/308, A61P 3/06

(54) **Pharmaceutical and nutritional compositions comprising phytosterols, folic acid, cyanocobalamine, pyridoxine and alimentary fibers**

(30) Priority: 15.05.2000 BR 0001794
(71) Applicant: Laboratorios Biosinética Ltda, 06767-030 Taboao da Serra (BR)
(72) Inventor: Penteado. Roberto Luiz Bruno, 01333-001 Sao Paulo (BR); Falci, Marcio, 05641-900 Sao Paulo (BR)
(74) Representative: Bianchetti, Giuseppe, Prof.

(57) **Abstract**

The present invention relates to pharmaceutical or nutritional compositions comprising phytosterols, folic acid, cyanocobalamine, pyridoxine and alimentary fibers for the treatment and prophylaxis of hypercholesterolemia and hyperhomocysteinemia.

## Description

The present invention relates to pharmaceutical or nutritional compositions comprising phytosterols, folic acid, cyanocobalamine, pyridoxine and alimentary fibers for the treatment and prophylaxis of hypercholesterolemia and hyperhomocysteinemia.

The compositions according to the invention decrease cholesterol and homocysteine concentration in blood and therefore may be used for the treatment and prevention of a number of pathological conditions such as infarction, brain hemorrhage, atherosclerosis, osteoporosis, occlusion of blood vessels, ocular modifications, neuropathies, and the like.

The pathogenic role of abnormal level of serum homocysteine is well known and discussed for instance in J. Am. Med. Ass., 1997, 1775-1781, 277(22).

The use folic acid, Vitamin B12 and B6 in treating elevated homocysteine levels has already been proposed, for instance in US 5,795,873.

Hypercholesterolemia is a specific risk factor for the evolution of atherosclerosis, and for one of its most serious complications, coronary insufficiency. In the same way, the biological characteristics and metabolism of cholesterol are well known. This molecule derives both from diet and from cellular synthesis, and is part of the normal cell structure, working as precursor for the synthesis of steroid hormones. It is transported from liver by body cells through the LDL fraction of blood lipoproteins. After the cytolysis process, it is taken back to liver by HDL to be excreted together with bile. About 60% of the excreted cholesterol will be reabsorbed by the small intestine and taken back to the liver by portal hepatic system, this time through the chilomicron fraction. 2/3 of all the cholesterol absorbed by the digestive tract derive from this source and the rest derives from alimentary sources. Hypercholesterolemia is due to the unbalance caused by the dysfunction of one of the steps of the metabolic process, depending on the type of dyslipidemia. Cholesterol will eventually accumulate in a pathological way on the vascular sub-endothelium, forming the atheromatous plaque.

Coronary insufficiency is the first cause of death in western world. In the United States, in 1996, 41% of all deaths were due to cardiovascular diseases, mainly of coronary kind. In this Country, it is deemed that most deaths for coronary disease affect individuals having total serum cholesterol between 180 and 250 mg/dl. Some studies showed that a 10% decrease in total cholesterol levels would result in a 22% reduction of incidence of coronary heart disease. One way to control the intake of cholesterol was the one established by the NCEP protocols (National Cholesterol Education Program).

In Brazil, statistical data are not so dramatic; however, Duncan et al. showed that in Porto Alegre, in a population having a cholesterol total average level of 202 mg/dl, 14.9% had a cholesterol level higher than 250 mg/dl. Nicolau et al. showed that 16% of the population of São José do Rio Preto had cholesterol levels higher than 240 mg/dl.

It has now been found that the addition of alimentary fibers and of phytosterols to folic acid and Vitamins B6 and B12 is particularly effective in decreasing cholesterolemia and homocysteinemia.

The present invention therefore relates to pharmaceutical and nutritional compositions comprising phytosterols, folic acid, cyanocobalamine, pyridoxine and alimentary fibers for the treatment and prophylaxis of hypercholesterolemia and hyperhomocysteinemia.

The present invention accordingly provides alimentary or pharmaceutical compositions comprising:
a. at least one phytosterol (or isomers thereof) selected from ergosterol, stigmasterol, betasitosterol, campesterol and betasitostanol;
b. folic acid or a salt thereof;
c. cyanocobalamine;
d. pyridoxine;
e. alimentary fibers.

### A. Phytosterols

Phytosterols are sterols of vegetal origin. These compounds are well known and may be obtained by a number of vegetal sources.

According to the invention, phytosterol and major isomers thereof - Ergosterol, Stigmasterol, Betasitosterol, Betasitostanol, Campesterol - may be used in the solid form or in the form of oily, alcoholic or aqueous solutions. Instead of pure phytosterols, the compositions of the invention may also comprise extracts containing said phytosterols.

The action of phytosteroids is related to the metabolism of the lipids. Phytosteroids by oral dosage are substantially not absorbed by the human intestines. They are largely similar to cholesterol, since they bind to the intestinal micelles that are not absorbed: this results in a lower absorption of cholesterol and in the corresponding reduction of chilomicrons. The reduction of the hepatic contents concurs to and stimulates the capture of LDL and the lower production of VLDL and apo B. Phytosteroids reduce hypercholesterolemia do not induce the side effects typical of other non-natural hypocholesterolemizing drugs. The oral administration of phytosteroids is advantageous also from the organo-leptic point of view and in terms of compliance.

### B. Folic acid

This vitamin is known to interfere in the biosynthesis of purine and thiamine, to participate in the synthesis of the methyl (-CH₃) group in the processes of homocysteine methylation for producing methionine, and in several growth processes, particularly in erythropoiesis.

Folic acid and vitamins B6 and B12 participate in the organic synthesis of methionine, an indispensable amino acid in the diet; it maintains the nitrogen balance for growth and life. Its deficiency interrupts growth, causes multiple problems, and may lead to death.

### C. Cyanocobalamine (Vitamin B12)

B12 vitamin participates in the metabolism of the methyl-labile group, particularly in the biosynthesis of methionine, by the transformation of homocysteine and choline through the participation of ethanolamine.

B12 vitamin participates in the metabolism and synthetic pathways of purines: among the metabolic reactions unequivocally dependent on B12 vitamin, reactions already well clarified are those of methylmalonyl-CoA mutase, that act on the isomeric conversion between methylmalonyl-CoA and succinyl-CoA, and above all the methylation of homocysteine into methionine, which produces methionine and tetrahydrofolate.

Whenever the procedure of methylation of homocysteine is not perfectly performed, a relative deficit of methionine will occur.

Studies performed in chicks and mice, administering homocysteine without the substances that provide the methyl (-CH₃) group, such as methionine, betaine and choline, have shown the occurrence of disruption of the growth process of the animals.

Through the supplementation of liver extract or of B12 vitamin, the corresponding growth was resumed. Therefore it has been evidenced that vitamin B12 participates in the methylation of homocysteine.

### D. Pyridoxine (Vitamin B6)

B6 vitamin acts as coenzyme involved in the transamination, deamination, decarboxylation, desulphydration and several biochemical transformations of amino acids. The normal metabolism of amino acids is of great importance to the disintoxication reactions. Vitamin B6 also takes part in the maintenance of a proper level of CoA in the liver. The metabolism of fatty acids is impaired in the absence of vitamin B6, resulting in problems with the metabolism of lipids.

In the metabolism of cysteine, the vitamin B6 reactions are related with the transfer of sulphur from methionine to serine, to give cysteine.

### E. Dietetic Fibers (Alimentary)

Examples of dietetic fibers for use in the compositions of the invention are cellulose, hemicellulose, pectin, lignin, gums, mucilages and algae polysaccharides.

The dietetic fibers correspond to the organic residues of foodstuff (animal or vegetal) that cannot be hydrolysed by the human digestive juices. The dietetic fibers are commercially available as cereal flakes, Musli, Granola® , All Bran® , biscuits, seed brans in general (wheat, oats, barley, rye, plantain, etc.), mucilages, alginates, autolyses of animal products and residues from the extraction of sugar from sugar beets.

The main function of cellulose in the intestines is to absorb water, one its gram of fibre being able to retain 400 mg of water.

At intestinal level hemicellulose is capable of retaining water and has the property of linking to cations.

Pectin produces gel by retaining water, and links to cations and organic matter, promoting the excretion of biliary acids.

Lignin, in the intestinal tract, inhibits microbial digestion of the cellular wall, by coating cellulose and hemicellulose, and may inhibit the division of the carbohydrates of the cellular wall. Lignin is capable of combining with biliary acids, forming non absorbing complexes (insoluble), reducing the levels of cholate in blood, and providing the transformation of hepatic cholesterol into biliary salts.

Gums and mucilages may form gel in the small intestine and bind to biliary acids and other organic matters. They promote the increase of volume of the faecal bolus and participate in the reduction of cholesterol, by changing the metabolism of salts.

Algae are complex polymers. The anaerobic fermentation of the polysaccharides results in energy for the development and preservation of the bacterial flora of the colon.

The compositions of the present invention provide an effective pharmacological tool to avoid the risk of infarction and brain vascular damage due to the progress of the atherosclerotic process and to age-related homocysteine, as well as the increase in endogenous homocysteine.

The compositions of the invention may be presented in the form of powder, sugar-coated pills, capsules, tablets, pastes, emulsions, granulates and may optionally contain other ingredients such as polyunsaturated fatty acids (Omega 3 and Omega 6) and bile acids. Depending on whether the compositions will be directed to a pharmaceutical, particularly as non-prescription formulation, or alimentary use (health food or nutraceutical), suitable excipients (e.g. preservants, sweetening agents, flavours, lubricants, etc.) and formulation methods will be used.

The compositions of the invention will preferably contain the ingredients in the following by weight percentages:

| | |
|---|---|
| Phytosterols | 1-5%; |
| folic acid | 0.00001-0.0001%, |
| vitamin B12 | 0.000001-0.0001%; |
| vitamin B6 | 0.1-0.01%; |
| Fibres | 10-60%. |

The compositions of the invention may be administered at daily doses ranging from 10 to 50 g, depending on the cholesterolemia and homocysteinemia levels, the age, weight and sex of the subject in need of treatment.

The following Examples illustrate the invention in more detail.

### Example 1:

| 100 g contain: | |
|---|---|
| Powder whole milk | 4.200g |
| Fibrex 608 | 26.100g |
| Beta-Sitosterol | 2.700g |
| Oat flakes | 2.473g |
| Rice crispies | 15.000g |
| Folic Acid | 0.001g |
| Vitamin B6 | 0.025g |
| Vitamin B 12 | 0.0005g |
| Maracuja pulp | 4.000g |
| Orange flavour 5119/D | 0.200g |
| Fumaric acid CWS | 0.010g |
| Salt | 0.010g |
| Glucose syrup | 45.000g |
| Liquid sugar | 10.000g |
| Soy Lecithin | 0.200g |
| Cocoa fat | 3.000g |

The formulation of example 1 has a prevention purpose.

### Example 2

| 100 g contain: | |
|---|---|
| Powder whole milk | 4.200g |
| Fibrex 608 | 33.000g |
| Beta-Sitosterol | 3.600g |
| Oat flakes | 0.001g |
| Rice Crispies | 9.700g |
| Folic Acid | 0.002g |
| Vitamin B6 | 0.050g |
| Vitamin B12 | 0.001g |
| Maracuja pulp | 4.000g |
| Orange flavour 5119/DP | 0.200g |
| Fumaric acid CWS | 0.010g |
| Salt | 0.010g |
| Glucose syrup | 45.000g |
| Liquid sugar | 10.000g |
| Soy Lecithin | 0.200g |
| Cocoa fat | 3.000g |

The formulation of example 2 has a treatment purpose.

These preparations are in the form of bars; a dry mixture of powder whole milk, Fibrex 608, beta-sitosterol, oat flakes, rice crispies, folic acid, vitamin B6, vitamin B12, Maracuja pulp, orange flavour 5119/DP, fumaric acid CWS and salt was added with a previously prepared mixture containing glucose syrup, liquid sugar, soy lecithin and cocoa fat, to form a homogeneous humid mass. The humid mass was then extruded through adequate equipment and formed in 25 g bars.

The concentrations of fibres, folic acid and vitamins B6 and B12 used in the formulation, as shown in examples 1 and 2, are as described before, the recommended daily dosage being a 25 g bar after the two main meals (lunch and dinner in most cases).

The formulations of examples 1 and 2 in the form of powder, sugar-coated pills, capsules, tablets, paste, various emulsions, granulates and emulsified concentrates do not exhibit any problems concerning uniformity or coherence of content of the active ingredients.

## Claims

1. Pharmaceutical or nutritional compositions comprising:
a. at least one phytosterol selected from ergosterol, stigmasterol, betasitosterol, campesterol and betasitostanol;
b. folic acid or a salt thereof;
c. cyanocobalamine;
d. pyridoxine;
e. alimentary fibers.

2. Compositions according to claim 1 comprising betasitosterol and betasitostanol.

3. Compositions according to claim 1 or 2, wherein the fibers are selected from cellulose, hemicellulose, pectin, lignin, gums, mucilages, algae polysaccharides.

4. Compositions according to any one of the previous claims in form of powder, sugar-coated pills, capsules, tablets, pastes, emulsions, granulates.
